# EUROPÄISCHE PATENTANMELDUNG

(11) **EP 3 357 978 A1**
(43) Veröffentlichungstag der Anmeldung: **08.08.2018**
(21) Anmeldenummer: 18155236.5
(22) Anmeldetag: 06.02.2018
(51) Int. Cl.: C09C 1/00, A23G 1/30, A23G 3/34, A61Q 1/00, C09D 17/00, A23L 5/42, C09D 7/40

(54) **FÄRBUNG VON OBERFLÄCHEN**

(30) Priorität: 07.02.2017 DE 102017001107
(71) Anmelder: Merck Patent GmbH, 64293 Darmstadt (DE)
(72) Erfinder: Schweinfurth, Ralf, 64287 Darmstadt (DE)

(57) **Zusammenfassung**

Die vorliegende Erfindung betrifft ein Verfahren zur Färbung von Oberflächen, insbesondere Oberflächen von Lebensmitteln, mit Effektpigmenten, vorzugsweise Perlglanzpigmenten, basierend auf plättchenförmigen Substraten, wobei (i) das Effektpigment in eine pastöse, transparente Masse auf Wasserbasis eingebracht und (ii) das in Schritt (i) hergestellte Auftragsmedium auf die Oberfläche aufgebracht wird.

## Beschreibung

Die vorliegende Erfindung betrifft ein Verfahren zur Färbung von Oberflächen, insbesondere Oberflächen von Lebensmitteln, mit Effektpigmenten basierend auf plättchenförmigen Substraten.

Außer für funktionelle Verwendungen werden Effektpigmente, wie z.B. Metall-, Perlglanz- und Interferenzpigmente, immer mehr zur optischen Aufwertung von Produkten, z. B. in der Kosmetik, eingesetzt, da schöne Farben und Effekte beim Betrachter und Konsumenten angenehme subjektive Empfindungen hervorrufen. Bei der Herstellung von Perlglanzpigmenten, z. B. für den Nahrungsmittelbereich, werden strengste Anforderungen an die Reinheit und die Qualität der Pigmente gestellt. Perlglanzpigmente werden im Nahrungsmittelbereich bereits zur Verbesserung des Farbeffektes bzw. zur Farbgebung eingesetzt.

Perlglanzpigmente werden auf unterschiedlichste Art und Weise auf Oberflächen, z.B. Oberflächen von Lebensmitteln aufgebracht oder in Lebensmittel eingearbeitet. Im Falle des Auftrags auf eine z.B. Lebensmittel-Oberfläche muss je nach gewünschtem Farbeffekt und Art des Lebensmittels das gegebenenfalls zum Aufbringen verwendete Auftragsmedium passend zum Lebensmittel ausgewählt werden. Unter dem Begriff Auftragsmedium ist hierbei in der Regel ein Fluid zu verstehen, in das das Perlglanzpigment zum Zwecke des Auftrags eingearbeitet ist. Auch die gegebenenfalls verwendete Auftragsmethode hat wesentlichen Einfluss auf Qualität und Quantität des Perlglanzeffektes. Oftmals ist die gewünschte Auftragsmethode nicht für das Lebensmittel geeignet, bzw. diese eignet sich nicht oder nur bedingt für das zur Färbung mögliche oder gewünschte Auftragsmedium.

Herkömmlicherweise werden Perlglanzpigmente z.B. durch Sprühverfahren und sich gegebenenfalls anschließende Trocknungsschritte auf Oberflächen aufgebracht. Diese Vorgehensweise ist aufwendig und kostenintensiv. Bei der Verwendung von Fettglasuren als Auftragsmedium für Perlglanzpigmente kann der verfahrensbedingte Reinigungsaufwand erheblich zu den Kosten bzw. Prozesszeiten beitragen. Werden Perlglanzpigmente direkt, d.h. ohne Verwendung eines Auftragsmediums, verwendet, besteht im Produktionsbereich das Risiko der Cross-Kontamination durch Pigmentstäube.

Aufgabe der vorliegenden Erfindung ist es daher, ein einfaches Verfahren zum Auftrag von Perlglanzpigmenten auf Oberflächen zur Verfügung zu stellen, welches zudem auf eine Vielzahl von Oberflächen, insbesondere Oberflächen unterschiedlichster Lebensmittel anwendbar ist und gleichzeitig das Spektrum der bestehenden Auftragsverfahren im Hinblick auf Wirtschaftlichkeit, optischen Eindruck der Oberfläche und Anwendbarkeit erweitert.

Überraschenderweise wurde gefunden, dass ein Perlglanzpigment auf eine Oberfläche vorteilhaft aufgebracht werden kann durch ein Verfahren, in welchem das Perlglanzpigment zunächst in ein pastöses, transparentes Auftragsmedium auf Wasserbasis eingebracht und diese Mischung anschließend auf die Oberfläche aufgebracht wird.

Bevorzugte Effektpigmente sind Perlglanzpigmente und Interferenzpigmente basierend auf plättchenförmigen Substraten, die mit ein oder mehreren Metalloxiden und/oder Metallhydroxiden beschichtet sind.

Unter dem Begriff plättchenförmige Substrate sind alle dem Fachmann bekannten plättchenförmigen Substrate zu verstehen. Geeignete Basissubstrate für die Effektpigmente, wie z.B. Perlglanzpigmente und Interferenzpigmente, sind transparente oder semitransparente plättchenförmige Substrate. Insbesondere geeignet sind Schichtsilikate, Talkum, Kaolin, plättchenförmige Eisen- oder Aluminiumoxide, Glas-, SiO₂-, TiO₂-Plättchen, plättchenförmige Mischoxide, wie z. B. FeTiO₃, Fe₂TiO₅, oder andere vergleichbare Materialien, abhängig von der jeweiligen gesetzlichen Zulässigkeit für die Verwendung bei Lebensmitteln.

Die Größe der Basissubstrate ist an sich nicht kritisch und kann auf den jeweiligen Anwendungszweck abgestimmt werden. In der Regel haben die plättchenförmigen Substrate eine Dicke von 0,005 bis 10 µm, insbesondere von 0,05 bis 5 µm. Die Ausdehnung in den beiden anderen Bereichen beträgt üblicherweise 1 bis 500 µm, vorzugsweise 2 bis 200 µm, und insbesondere 5 bis 150 µm. Ganz besonders bevorzugte Perlglanzpigmente weisen Partikelgrößen von 10-60 µm oder 5-25 µm oder 10-150 µm oder 5-50 µm auf.

Bevorzugte Effektpigmente, wie z.B. Perlglanzpigmente, basieren auf natürlichen Glimmerplättchen, synthetischen Glimmerplättchen, SiO₂-Plättchen, Al₂O₃-Plättchen, TiO₂-Plättchen, Glasplättchen, Fe₂O₃-Plättchen, insbesondere synthetischen Glimmerplättchen, natürlichen Glimmerplättchen und SiO₂-Plättchen (Nr. E555 bzw. E551 in der Liste der in der Europäischen Union zugelassenen Lebensmittelzusatzstoffe). Die synthetischen Plättchen, wie z.B. synthetische Glimmerplättchen, SiO₂-Plättchen, Al₂O₃-Plättchen, TiO₂-Plättchen, Glasplättchen, können dotiert oder undotiert sein. Geeignete Dotiermittel sind u.a. Metalloxide, wie z.B. TiO₂, ZrO₂, SnO₂.

Als Perlglanzpigmente werden Pigmente auf der Basis plättchenförmiger, transparenter oder semitransparenter Substrate aus z.B. Schichtsilikaten, wie etwa natürlichem Glimmer, synthetischem Glimmer, Talkum, Sericit, Kaolin, aus Glas oder anderen silikatischen Materialien verwendet, die mit farbigen oder farblosen Metalloxiden wie z.B. TiO₂, Titansuboxide, Titanoxinitride, Fe₂O₃, Fe₃O₄, SnO₂, ZnO und anderen Metalloxiden allein oder in Mischung in einer einheitlichen Schicht oder in aufeinanderfolgenden Schichten beschichtet sind. Besonders bevorzugte Perlglanzpigmente basierend auf synthetischen oder natürlichen Glimmerplättchen, sind mit ein oder mehreren Metalloxiden aus der Gruppe TiO₂, Fe₂O₃ oder Fe₃O₄ oder deren Gemische beschichtet oder weisen eine Multilayer-Beschichtung auf bestehend aus alternierend hoch-und niedrigbrechenden Schichten, wie z.B. TiO₂ - SiO₂ - TiO₂.

Weiterhin bevorzugt sind mit TiO₂ und/oder Fe₂O₃ beschichtete SiO₂- oder Al₂O₃-Plättchen. Die Beschichtung der SiO₂-Plättchen mit ein oder mehreren Metalloxiden kann z.B. erfolgen wie in der WO 93/08237 (naßchemische Beschichtung) oder DE-OS 196 14 637 (CVD-Verfahren) beschrieben.

Auch bei den auf die plättchenförmigen Substrate aufgebrachten Metalloxiden handelt es sich vorzugsweise um für den Menschen verzehrbare Materialien in Lebensmittelqualität (Nr. E 171 (TiO₂) bzw. E 172 (Eisenoxid) in der Liste der in der Europäischen Union zugelassenen Lebensmittelzusatzstoffe).

Weiterhin sind bevorzugt Perlglanzpigmentgemische, die unterschiedliche Partikelgrößen aufweisen. Besondere Effekte können erzielt werden, wenn Perlglanzpigmente mit "kleinen" Partikelgrößen wie z.B. 5-25 µm oder 10-60 µm mit Perlglanzpigmenten mit "großen" Partikelgrößen, wie z.B. 10-150 µm, gemischt werden.

Die Dicke der einzelnen Schichten, vorzugsweise ein oder mehrere Metalloxidschichten, auf dem Basissubstrat beträgt vorzugsweise 10-500 nm, insbesondere 20-400 nm und ganz besonders bevorzugt 30-350 nm.

Bei Mehrschichtpigmenten, die auf der Substratoberfläche vorzugsweise alternierend hoch- und niedrigbrechende Schichten (A)(B)(A) aufweisen, besitzt die hochbrechende Schicht (Schicht A) in der Regel Schichtdicken von 10 - 500 nm, vorzugsweise 20 - 400 nm und insbesondere 30 - 350 nm. Die Dicke der niedrigbrechenden Schicht (Schicht B) beträgt vorzugsweise 10-500 nm, vorzugsweise 20 - 400 nm, insbesondere 30 - 350 nm.

Unter hochbrechenden Schichten sind in dieser Anmeldung Schichten mit einem Brechungsindex von ≥ 1,8 zu verstehen, wie z.B. TiO₂, Fe₂O₃, Fe₃O₄, während niedrigbrechende Schichten einen Brechungsindex von < 1,8 aufweisen, wie z.B. SiO₂, Al₂O₃. AlO(OH).

Die Effektpigmente, vorzugsweise Perlglanzpigmente, können mehrere, gleiche oder verschiedene Kombinationen an Schichtpaketen enthalten, bevorzugt ist aber die Belegung des Substrats bei Mehrschichtpigmenten mit nur einem Schichtpaket (A) (B) (A). Zur Intensivierung der Farbstärke kann das erfindungsgemäße Pigment bis zu 4 Schichtpakete enthalten, wobei die Dicke aller Schichten auf dem Substrat 3 µm allerdings nicht überschreiten sollte. Vorzugsweise wird bei Mehrschichtpigmenten mit 3 oder mehr Schichten auf der Substratoberfläche eine ungerade Anzahl von Schichten auf das plättchenförmige Substrat aufgebracht mit je einer hochbrechenden Schicht in innerster und äußerster Lage. Besonders bevorzugt ist ein Aufbau von drei optischen Interferenzschichten in der Reihenfolge (A) (B) (A). Als hochbrechende Schicht kommen vorzugsweise TiO₂, Fe₂O₃ und/oder Fe₃O₄ oder ein Gemisch aus Titanoxid und Eisenoxid in Frage. Das TiO₂ kann dabei in der Rutil- oder in der Anatasmodifikation vorliegen.

Als farblose niedrigbrechende für die Beschichtung (B) geeignete Materialien sind vorzugsweise Metalloxide bzw. die entsprechenden Oxidhydrate, wie z. B. SiO₂, Al₂O₃, AlO(OH), B₂O₃, MgF₂, MgSiO₃ oder ein Gemisch der genannten Metalloxide, geeignet entsprechend der gesetzlichen Zulassungen zur Verwendung bei Lebensmitteln bzw. pharmazeutischen Produkten.

Bei den besonders bevorzugten Perlglanzpigmenten für den Lebensmittelbreich handelt es sich um Glimmer-Plättchen (synthetisch oder natürlich) oder SiO₂-Plättchen, die mit einer Metalloxidschicht (TiO₂ oder Fe₂O₃ oder Fe₃O₄) der Dicke von 10 nm bis 500 nm umhüllt sind. Die Dicken der Plättchen liegen im Bereich von 200 nm bis 900 nm. Derartige Pigmente zeichnen sich je nach Dicke der eingesetzten Plättchen und der aufgebrachten Metalloxidschichten sowie nach Art des Metalloxids durch besonders intensive Interferenzfarben und/oder durch starke winkelabhängige Farbwechseleffekte aus. Letztere zeigen sich dabei so, dass ein Betrachter beim Wechsel seiner Beobachtungsposition zum pigmentierten Objekt unterschiedliche Farben wahrnimmt.

Bevorzugte Effektpigmente sind insbesondere ausgewählt aus den nachfolgend genannten Pigmenten:
natürliche Glimmerplättchen + TiO₂
natürliche Glimmerplättchen + Fe₂O₃
natürliche Glimmerplättchen + Fe₃O₄
natürliche Glimmerplättchen + TiO₂ + Fe₂O₃
natürliche Glimmerplättchen + TiO₂ + Fe₃O₄
natürliche Glimmerplättchen + Fe₂O₃ + TiO₂
natürliche Glimmerplättchen + Fe₃O₄ + TiO₂
natürliche Glimmerplättchen + TiO₂/Fe₂O₃-Gemisch
natürliche Glimmerplättchen + TiO₂/Fe₃O₄-Gemisch
synthetische Glimmerplättchen + TiO₂
synthetische Glimmerplättchen + Fe₂O₃
synthetische Glimmerplättchen + Fe₃O₄
synthetische Glimmerplättchen + TiO₂ + Fe₂O₃
synthetische Glimmerplättchen + TiO₂ + Fe₃O₄
synthetische Glimmerplättchen + Fe₂O₃ + TiO₂
synthetische Glimmerplättchen + Fe₃O₄ + TiO₂
synthetische Glimmerplättchen + TiO₂/Fe₂O₃-Gemisch
synthetische Glimmerplättchen + TiO₂/Fe₃O₄-Gemisch
SiO₂-Plättchen + TiO₂
SiO₂-Plättchen + Fe₂O₃
SiO₂-Plättchen + Fe₃O₄
SiO₂-Plättchen + TiO₂ + Fe₂O₃
SiO₂-Plättchen + TiO₂ + Fe₃O₄
SiO₂-Plättchen + Fe₂O₃ + TiO₂
SiO₂-Plättchen + Fe₃O₄ + TiO₂
SiO₂-Plättchen + TiO₂/Fe₂O₃-Gemisch
SiO₂-Plättchen + TiO₂/Fe₃O₄-Gemisch
natürliche Glimmerplättchen + TiO₂ + SiO₂ + TiO₂
natürliche Glimmerplättchen + Fe₂O₃ + SiO₂ + TiO₂
natürliche Glimmerplättchen + TiO₂ + SiO₂ + Fe₂O₃
natürliche Glimmerplättchen + TiO₂ + SiO₂ + Fe₃O₄
natürliche Glimmerplättchen + TiO₂/Fe₂O₃-Gemisch + SiO₂ + Fe₂O₃
natürliche Glimmerplättchen + TiO₂/Fe₂O₃-Gemisch + SiO₂ + TiO₂/Fe₂O₃-Gemisch
natürliche Glimmerplättchen + Fe₂O₃-Gemisch + SiO₂ + TiO₂/Fe₂O₃-Gemisch
synthetische Glimmerplättchen + TiO₂ + SiO₂ + TiO₂
synthetische Glimmerplättchen + Fe₂O₃ + SiO₂ + TiO₂
synthetische Glimmerplättchen + TiO₂ + SiO₂ + Fe₂O₃
synthetische Glimmerplättchen + TiO₂ + SiO₂ + Fe₃O₄
synthetische Glimmerplättchen + TiO₂/Fe₂O₃-Gemisch + SiO₂ + Fe₂O₃
synthetische Glimmerplättchen + TiO₂/Fe₂O₃-Gemisch + SiO₂ + TiO₂/Fe₂O₃-Gem isch
synthetische Glimmerplättchen + Fe₂O₃-Gemisch + SiO₂ + TiO₂/Fe₂O₃-Gemisch
SiO₂-Plättchen + TiO₂ + SiO₂ + TiO₂
SiO₂-Plättchen + Fe₂O₃ + SiO₂ + TiO₂
SiO₂-Plättchen + TiO₂ + SiO₂ + Fe₂O₃
SiO₂-Plättchen + TiO₂ + SiO₂ + Fe₃O₄
SiO₂-Plättchen + TiO₂/Fe₂O₃-Gemisch + SiO₂ + Fe₂O₃
SiO₂-Plättchen + TiO₂/Fe₂O₃-Gemisch + SiO₂ + TiO₂/Fe₂O₃-Gemisch
SiO₂-Plättchen + Fe₂O₃-Gemisch + SiO₂ + TiO₂/Fe₂O₃-Gemisch.

Das Perlglanzpigment oder eine Mischung von Perlglanzpigmenten wird vorzugsweise in einer Menge von 0,05 - 45 Gew.-%, insbesondere 0,5 - 25 Gew.-%, besonders bevorzugt 5 - 15 Gew.-%, bezogen auf die Gesamtmasse des Auftragsmediums, eingesetzt.

Das Auftragsmedium enthält neben Wasser und Perlglanzpigment vorzugsweise einen Stabilisator (S), der dazu dient, das Effektpigment, vorzugsweise das Perlglanzpigment, in der Mischung in Suspension zu halten, das heißt dessen Sedimentation zu verhindern.

Stabilisatoren sind in einer großen Vielfalt bekannt und werden als solche angewandt. Eine Gruppe möglicher Stabilisatoren umfasst eine Kombination von kolloidaler mikrokristalliner Cellulose (MCC) sowie Carboxymethylcellulose (CMC). Als Stabilisatoren einsetzbar sind insbesondere die folgenden der Liste der in der Europäischen Union zugelassenen Lebensmittelzusatzstoffe: E322, E401, E402, E403, E404, E 405, E406, E407, E 410, E412, E413, E 414, E415, E 416, E417, E418, E422, E 423, E424, E425, E 426, E 440, E 445, E 460, E 463, E464, E 465, E 466, E458, E 469, E 499, E551, E553, E554, E 1201, E 1202, E1404, E 1410, E 1412, E 1413, E1414, E 1420, E1422, E 1440, E 1442, E 1450, E1520. Erfindungsgemäß bevorzugt wird E 466 (Natrium-Carboxymethylcellulose).

Der Stabilisator wird vorzugsweise in einer Menge von 0,25 - 8 Gew.-%, besonders bevorzugt 0,5 - 4 Gew.-%, ganz besonders bevorzugt 1,0 - 3,0 Gew.-%, bezogen auf die Gesamtmasse des Auftragsmediums, eingesetzt.

Weiterhin enthält das Auftragsmedium vorzugsweise einen Feuchteregulator (F), der ein schnelles und gleichmäßiges Antrocknen und Abtrocknen des Auftragsmediums an der Oberfläche bewirkt.

Feuchteregulatoren sind in einer großen Vielfalt bekannt und werden als solche angewandt. Als Feuchteregulatoren einsetzbar sind u.a. die folgenden der Liste der in der Europäischen Union zugelassenen Lebensmittelzusatzstoffe: E 420, E 422, E 964, E 968, E 1200, E 1520. Erfindungsgemäß bevorzugt wird E 1520 (1,2-Propandiol).

Der Feuchteregulator wird vorzugsweise in einer Menge von 0,5 - 25 Gew.-%, besonders bevorzugt 2,0 - 15 Gew.-%, ganz besonders bevorzugt 3,0 - 11,0 Gew.-%, bezogen auf die Gesamtmasse des Auftragsmediums, eingesetzt.

Weiterhin enthält das Auftragsmedium vorzugsweise einen Emulgator (E), der für homogene Mischung der im Auftragsmedium präsenten Komponenten sorgt.

Emulgatoren sind in einer großen Vielfalt bekannt und werden als solche angewandt. Als Emulgatoren einsetzbar sind u.a. die folgenden der Liste der in der Europäischen Union zugelassenen Lebensmittelzusatzstoffe: E 322, E 405, E 426, E 432, E 442, E 444, E 459, E 470a, E 471, E 472b, E 472e, E 473, E 476, E 481, E 483, E 491, E 920. Erfindungsgemäß bevorzugt wird E 322 (Soja- oder Sonnenblumen-Lecithin).

Der Emulgator wird vorzugsweise in einer Menge von 0,25 - 5 Gew.-%, besonders bevorzugt 0,5 - 3 Gew.-%, ganz besonders bevorzugt 1,0 - 2,0 Gew.-%, bezogen auf die Gesamtmasse des Auftragsmediums, eingesetzt.

Weiterhin enthält das Auftragsmedium vorzugsweise einen Konservierungsstoff (K), der für mikrobiologische Stabilität der Auftragsmedium-Mischung sorgt.

Konservierungsstoffe sind in einer großen Vielfalt bekannt und werden als solche angewandt. Als Konservierungsstoff einsetzbar sind u.a. die folgenden der Liste der in der Europäischen Union zugelassenen Lebensmittelzusatzstoffe: E 200, E 202, E 203, E 210, E 211, E 212, E 213, E 214, E 215, E218, E 219, E220, E221, E 222, E 223, E 224, E 226, E 228, E 231, E 232, E 234, E 235, E 239, E 242, E 249, E 250, E 251, E 252, E 260, E 261, E 262, E 263, E 280, E 281, E 282, E 283, E 284, E 285, E 1105, E 1519. Erfindungsgemäß bevorzugt werden als Konservierungsstoff Kombinationen von E 202 mit Lebensmittelsäuren wie z.B. E 330, E 270 oder anderen Lebensmittelsäuren.

Das Konservierungsstoff-Gemisch wird vorzugsweise in einer Menge von 0,1 - 1,5 Gew.-%, bevorzugt 0,2 - 1 Gew.-%, besonders bevorzugt 0,25 - 0,5 Gew.-%, bezogen auf die Gesamtmasse des Auftragsmediums, eingesetzt. Das Konservierungsstoff-Gemisch besteht typischerweise zu 75 - 90 Gew.-% aus Lebensmittelsäure und zu 10-25 Gew.-% aus dem eigentlichen Konservierungsstoff.

Optional können der Auftragsmedium-Mischung weitere Komponenten (W), wie z.B. Süßstoffe, Aromastoffe oder andere Farbstoffe zugegeben werden.

In einer bevorzugten Ausführungsform enthält das Auftragsmedium Wasser + S + F + E + K + Effektpigment, vorzugsweise mindestens ein Perlglanzpigment, + optional W.

In einer weiteren Ausführungsform der Erfindung kann die Auftragsmedium-Mischung weiter mit Wasser verdünnt werden, bzw. bereits in der Grundrezeptur ein Teil des Wassers durch Ethanol ersetzt werden. Durch letztere Verfahrensweise wird die Trocknungszeit des aufgetragenen Auftragsmediums verkürzt und die mikrobiologische Stabilität der Auftragsmedium-Mischung erhöht.

Zur Herstellung der Auftragsmedium-Mischung können herkömmliche Mischverfahren und Rührer, typischerweise unter Verwendung eines offenen Gefäßes eingesetzt werden. In das mit Wasser beschickte Gefäß werden die Komponenten S, F, E, K, sofern jeweils vorhanden, und gegebenenfalls W der Auftragsmedium-Mischung in beliebiger Reihenfolge oder alle gleichzeitig eingegeben und anschließend durch Rühren vermischt. Optional kann der Mischprozess durch Erwärmen beschleunigt werden.

In die hergestellte wasserbasierte Mischung der Komponenten S, F, E, K und gegebenenfalls W werden nun die Perlglanzpigmente einzeln, Kombinationen von Perlglanzpigmenten, oder Kombinationen der beiden vorgenannten Optionen mit weiteren Farbstoffen/Pigmenten, wie z.B. rote Beete, ß-Carotin, Paprika E160c, Anthocyane, Karamell, Pflanzenkohle, Karmin E120, Riboflavin, Zuckerkulör E150, Kurkumin, etc., mineralischen (Eisenoxid, Titandioxid, etc.) oder künstlichen Lebensmittelfarbstoffen (E 131, E 129, E 133, E 127, E 141, E 102, E 122, E 124, E 110, E123, E132, E 104, etc.) unter weiterem Rühren hinzugefügt. Gleiches gilt auch für die Verwendung in Kombination mit sogenannten färbenden Pflanzen-und Fruchtextrakten, färbenden Lebensmitteln (wie z.B. schwarze Karotte, Brennnessel, Spirulina, etc.). Aromen jeglicher Art und Süßstoffe, wie z.B. Aspartam, Acesulfam K, Stevia, Saccharin, Cyclamat, etc., können nach Wunsch dem Medium hinzugefügt werden

Das Auftragsmedium kann mittels einfacher Verfahren, z.B. per Handauftrag, auf Oberflächen, insbesondere Oberflächen von Lebensmitteln aufgetragen werden. Das Auftragsmedium kann auch ohne vorherige Beschichtung als Dekoration nach entsprechender Trocknung auf Oberflächen verwendet werden. Hierzu wird es zuvor mittels entsprechender Formen oder Unterlagen in die gewünschte Form gebracht.

Das erfindungsgemäße, einfach herstellbare und ausschließlich aus lebensmittelrechtlich zugelassenen Komponenten bestehende Auftragsmedium kann zur Einfärbung von Oberflächen insbesondere einer Vielzahl verschiedenster Lebensmittel verwendet werden.

Perlglanzpigmente können in einem breiten Konzentrationsbereich von 0,05 - 45 Gew.-% im Auftragsmedium enthalten sein. Durch die vorliegende Erfindung sind neuartige und intensivere Perlglanzeffekte möglich. Produkte, welche bisher nur schwer oder ohne zufriedenstellenden Effekt mit Perlglanzpigmenten gefärbt werden konnten, können mittels der vorliegenden Erfindung gefärbt werden. Es erfolgt keine Sedimentation der Perlglanzpigmente nach der Einarbeitung in das Auftragsmedium. Perlglanzpigmente können mit anderen Farbstoffen/Pigmenten (wie z.B. natürlich färbende Pflanzenextrakte, künstliche Farbstoffe) kombiniert werden. Möglich ist auch die individuelle Zugabe von Süßstoffen und Aromen jedweder Art. Das Auftragsmedium kann mittels einfacher Methoden, wie z.B. per Handauftrag, auf die Produkte aufgetragen werden. Durch die Vermeidung von Perlglanzstaub während des Färbeprozesses kommt es zu keiner Kontamination.

Die nachfolgenden Beispiele sollen die Erfindung erläutern, ohne sie jedoch zu begrenzen. Sofern nicht anders angegeben sind alle Prozentangaben in dieser Anmeldung Gewichtsprozent.

### Beispiele:

### Beispiel 1: Färbung von hart gekochten Eiern

### Auftragsmedium:

| | | |
|---|---|---|
| 80 | Gew.-% Wasser | |
| 5,75 | Gew.-% 1,2 Propandiol E1520 | Merck KGaA |
| 2,4 | Gew.-% Natriumcarboxymethylcellulose | Roeper |
| 1,5 | Gew.-% Soja- bzw. Sonnenblumen-Lecithin | Sternchemie |
| 0,3 | Gew.-% Zitronensäure (kristallin) | Merck KGaA |
| 0,05 | Gew.-% Kaliumsorbat | Merck KGaA |
| 10 | Gew.-% Perlglanzpigment (A1 - A11) | Merck KGaA |

A1) Candurin® Gold Lustre (Glimmer + Titandioxid + Eisenoxid)
A2) Candurin® Brown Amber (Glimmer + Eisenoxid)
A3) Candurin® Gold Shimmer (Glimmer + Titandioxid)
A4) Candurin® Silver Lustre (Glimmer + Titandioxid)
A5) Candurin® Silver Lustre + Candurin® Gold Lustre (9 : 1 Gew.-%)
A6) Candurin® Silver Lustre + E131 (Gewichtsverhältnis: 0,005 : 0,5)
A7) Candurin® Silver Lustre + E153 oder E172 schwarz (Gewichtsverhältnis: 0,01 : 1)
A8) Candurin® NXT Ruby Red: (Siliziumdioxid + Eisenoxid)
A9) Candurin® Red Lustre (Glimmer + Eisenoxid)
A10) Candurin® Silver Lustre + Candurin® Red Lustre (Gewichtsverhältnis: 8 : 2)
A11) Candurin® Silver Lustre + Candurin® Silver Sparkle (Gewichtsverhältnis: 8 : 2), beide Glimmer + Titandioxid: Silver Sparkle mit größerem Partikeldurchmesser

### Herstellung des Auftragsmediums

Wasser wird mit Kaliumsorbat und Zitronensäure vorgelegt, mit einem Flügel-Rührer gerührt und die Mischung auf maximal 80 °C erhitzt. Während des Erhitzens wird Natriumcarboxymethylcellulose unter ständigem Rühren hinzugegeben. Es wird so lange gerührt (ca. 45 min.) bis sich die gesamte Cellulose gelöst hat. Alternativ kann das Gemisch ohne Wärmezufuhr für 3 h gerührt werden.

Sobald sich die gesamte Natriumcarboxymethylcellulose im Wasser gelöst hat, werden 1,2-Propandiol und Lecithin bei Raumtemperatur hinzu gegeben, und weiter gerührt bis sich alle Inhaltsstoffe gleichmäßig verteilt haben. Im Anschluss wird die angegebene Pigmentmenge untergerührt.

### Färbung

Eier werden hart gekocht und im Anschluss mittels eines erfindungsgemäßen Auftragsmediums gefärbt. Hierzu wird eine kleine Menge des Mediums auf einen Handschuh gegeben. Auch die andere Hand trägt hierbei einen Handschuh. Das gekochte Ei sollte warm und trocken sein. Durch schnelles hin und her-Reiben in den Händen wird das Auftragsmediums gleichmäßig auf der warmen Eierschale verteilt. Bedingt durch die Restwärme des Eis trocknet das Auftragsmedium schnell ab. Man erhält eine sehr gleichmäßige Perlglanzeinfärbung. Die Eier können je nach ausgewähltem Perlglanzpigment weiß- oder braunschalig sein.

Abweichende Perlglanzpigmentmengen von 2-17 Gew.-% führen zu unterschiedlichen Intensitäten des resultierenden Perlglanzeffektes.

Es kommt zu keiner Farbpenetration durch die Eischale.

### Beispiel 2: Schokolade und Pralinen

### Beispiel 2.1: Fertige Pralinen, Schokoladen und schokoladeüberzogene Produkte im Allgemeinen können mit dem Perlglanzpigment-Auftragsmedium nachträglich verziert bzw. dekoriert werden.

### Auftragsmedium:

| | | |
|---|---|---|
| 60 | Gew.-% Wasser | |
| 20 | Gew.-% Ethanol (96 Vol. %) | Merck KGaA |
| 5,75 | Gew.-% 1,2-Propandiol E1520 | Merck KGaA |
| 2,4 | Gew.-% Natriumcarboxymethylcellulose | Roeper |
| 1,5 | Gew.-% Soja- bzw. Sonnenblumen-Lecithin | Sternchemie |
| 0,3 | Gew.-% Zitronensäure (kristallin) | Merck KGaA |
| 0,05 | Gew.-% Kaliumsorbat | Merck KGaA |
| 10 | Gew.-% Perlglanzpigment (ausgewählt aus A1) -A11) gemäß Beispiel 1) | Merck KGaA + optional Süßstoff und/oder Aromen nachWahl |

### Herstellung des Auftragsmediums wie in Beispiel 1

### Färbung:

Das Auftragsmedium wird mittels feiner Düse, Spatel oder mit einem Pinsel auf die zu färbenden Stellen des Schokoladenproduktes aufgebracht. Danach wird bei Raumtemperatur getrocknet, bis der Farbüberzug vollständig trocken ist.

Mittels einer zusätzlichen Wasser-Zugabe kann die Viskosität des Mediums herabgesetzt werden. Dadurch lässt sich das Medium ggf. besser auf bestimmte Produkt-Stellen auftragen. Die Trocknungszeit wird dadurch erhöht.

### Beispiel 2.2: Vorfärbung von Schokolade-Formen

### Auftragsmedium:

| | | |
|---|---|---|
| 80 | Gew.-% Wasser | |
| 10,75 | Gew.-% 1,2 Propandiol E1520 | Merck KGaA |
| 2,4 | Gew.-% Natriumcarboxymethylcellulose | Roeper |
| 1,5 | Gew.-% Soja- bzw. Sonnenblumen-Lecithin | Sternchemie |
| 0,3 | Gew.-% Zitronensäure (kristallin) | Merck KGaA |
| 0,05 | Gew.-% Kaliumsorbat | Merck KGaA |
| + | Perlglanzpigment (ausgewählt aus A1) -A11) gemäß Beispiel 1) | Merck KGaA |

### Herstellung des Auftragsmediums:

Die Komponenten werden wie in Beispiel 1 beschrieben vermischt. Vor Zugabe des Pigments werden weitere 50 Gew.-% Wasser zugegeben und weiter gerührt, bis sich alles homogen verteilt hat

Zu dieser Mischung werden 5 Gew.-% des ausgewählten Perlglanzpigments gemäß A1) bis A11) hinzugegeben.

### Färbung:

Schokoladeformen aus Kunststoff (z.B. Polycarbonat) oder Metall lassen sich sehr einfach unter Verwendung des erfindungsgemäßen Auftragsmediums vordekorieren. Hierbei wird das Pigment-haltige Auftragsmedium in die Form auf die gewünschten Stellen gegeben. Anschließend wird die Form in einem Umluftofen oder Trockenschrank getrocknet ist. Die Färbung der Form kann durch geeignete Düsen, Pipetten, Pinsel oder per Hand durchgeführt werden.

Mittels einer zusätzlichen Wasser-Zugabe kann die Viskosität des Mediums herabgesetzt werden. Dadurch lässt sich das Medium ggf. besser auf bestimmte Produkt-Stellen auftragen. Die Trocknungszeit wird dadurch erhöht.

Optional kann ein Teil des Wassers bei der Herstellung des Auftragsmediums durch Ethanol (Lebensmittelqualität 80 - 99,99 Vol-%) ersetzt werden. Dadurch wird die Abtrocknung beschleunigt. Es können 5 - 30 % des Wassers durch Ethanol ersetzt werden.

### Beispiel 2.3: Weiterhin ist es möglich, Schokoladeprodukte analog zu Beispiel 1 per Hand gleichmäßig zu färben.

### Beispiel 3: Dekoration anderer Süßwaren

Analog zur Dekoration von Schokolade und Pralinen (Beispiel 2) können auch andere Süßwaren wie z.B. Marzipan oder Fondantartikel, nachträglich mit dem erfindungsgemäßen Auftragsmedium dekoriert bzw. verziert werden.

Diese Produkte können im Anschluss an den Auftrag im Gegensatz zu Schokolade und Pralinen bei höheren Temperaturen getrocknet werden.

Ansonsten gelten die Rezeptur-Variationen, Herstellverfahren und Färbungsmöglichkeiten wie bei Beispiel 2 beschrieben.

### Beispiel 4: Dekoration, Färbung von Cerealien

Extrudierte Frühstückscerealien lassen sich nur schwer mit den üblichen Perlglanzauftragsmethoden färben. Ein Auftrag mittels einer verdünnten Zuckerlösung führt noch zu den besten Ergebnissen, doch wird dadurch auch der Zuckeranteil im Produkt erhöht. Außerdem kann kristallisierender Zucker zu einer Verringerung des Perlglanzeffektes führen.

Mittels des entwickelten Perlglanz-haltigen Auftragsmediums lassen sich auch Cerealien problemlos einfärben.

### Auftragsmedium (Basis):

| | | |
|---|---|---|
| 70 | Gew.-% Wasser | |
| 23 | Gew.-% Ethanol (96vol %) | Merck KGaA |
| 3,5 | Gew.-% 1,2 Propandiol E1520 | Merck KGaA |
| 2,0 | Gew.-% Natriumcarboxymethylcellulose | Roeper |
| 1,2 | Gew.-% Soja- bzw. Sonnenblumen-Lecithin | Sternchemie |
| 0,25 | Gew.-% Zitronensäure (kristallin) | Merck KGaA |
| 0,05 | Gew.-% Kaliumsorbat | Merck KGaA |

### Optional Süßstoffe & Aromen nach Wahl

Dieser wie in Beispiel 1 hergestellten Basis werden dann je nach gewünschtem Perlglanzeffekt die entsprechenden Perlglanzpigmente, einzeln oder in Kombination bzw. zusätzlich andere Farbstoffe oder färbende Frucht und Pflanzenextrakte hinzugefügt.

### Färbung:

### Beispiel 4.1: Schokocerealien, rund, extrudiert

Cerealien werden in einen Kessel gegeben. Dieser ist mit Mitnehmern ausgestattet. Trocknungsluft ist eingeschaltet (40 - 50°C)

### Cerealien-Einwaage: 40 g

Auftragsmedium:

| | | |
|---|---|---|
| 10 | Gew.-% Candurin® Silver Lustre | Merck KGaA |
| 0,05 | Gew.-% Pflanzenkohle | CHR Hansen |
| 89,95 | Gew.-% Auftragsmedium Basis (Herstellung wie oben) | |

Nach und nach werden insgesamt 60 g (= 15 % der Cerealienmenge) an Auftragsmedium auf die im Kessel rotierenden Cerealien aufgetragen. Hierbei werden 10 g des Auftragsmediums / Auftrag aufgegeben. Dieser verteilt sich auf den Cerealien. Trocknungsluft wird auf die Cerealien geleitet. Sobald ein Auftrag getrocknet ist, kann erneut eine entsprechende Auftragsmenge auf die Cerealien aufgebracht werden, bis der gewünschte Farbeffekt erzielt ist. Im Anschluss werden die Cerealien z.B. in einem Trockenschrank oder Ofen getrocknet bis die Ausgangsfeuchtigkeit wieder erreicht wird.

### Beispiel 4.2: Schokocerealien, rund, extrudiert

Es wird wie in Beispiel 4.1 verfahren.

| | | |
|---|---|---|
| Cerealien-Einwaage: | 400 g | |
| | | |
| Auftragsmedium: | 5 Gew.-% Candurin® Gold Lustre | Merck KGaA |
| | 3 Gew.-% Candurin® Gold Sparkle | Merck KGaA |
| | 92 Gew.-% Auftragsmedium Basis (Herstellung wie oben) | |
| | | |
| Auftragsmenge: | 50 g (= 12,5 %) | |

### Beispiel 4.3: Getreidecerealien, länglich, extrudiert

Es wird wie in Beispiel 4.1 verfahren.

| | | |
|---|---|---|
| Cerealien-Einwaage: | 500 g | |
| | | |
| Auftragsmedium: | 12% Candurin® NXT Ruby Red | Merck KGaA |
| | 88% Auftragsmedium Basis (Herstellung wie oben) | |
| | | |
| Auftragsmenge: | 40 g (= 8%) | |

### Beispiel 5: Färbung, Dekoration von Popcorn

Als mögliche (herkömmliche) Option kommt die Färbung des Popcorns über den zur Popcorn-Herstellung eingesetzten Zucker bzw. des verwendeten Fetts in Betracht. Die schnelle Karamellisierung des Zuckers würde jedoch zu einer Überdeckung des Perlglanzeffektes führen. Salziges Popcorn wäre herkömmlich über das zur Popcorn-Herstellung eingesetzte Fett einfärbbar. Die Einfärbung via Fett funktioniert auch nur zufriedenstellend bei verschlossenem Mikrowellen-Popcorn, allerdings sind die erforderlichen Einsatzmengen an Pigment sehr groß.

Analog zu Cerealien lässt sich Popcorn einfach mittels des erfindungsgemäßen transparenten Auftragsmediums mit Perlglanzpigmenten färben bzw. dekorieren.

### Beispiel 5.1: Popcorn gezuckert

| | | |
|---|---|---|
| Popcorn-Einwaage: | 300 g | |
| | | |
| Auftragsmedium: | 5 Gew.-% Candurin® Red Lustre | Merck KGaA |
| | 3 Gew.-% Candurin® Red Sparkle | Merck KGaA |
| | 92 Gew.-% Auftragsmedium Basis (Zusammensetzung und Herstellung wie in Beispiel 4) | |
| | | |
| Auftragsmenge: | 50 g (= 16,67 %) | |

Vom Auftragsmedium werden nach und nach insgesamt 50 g (= 16,67 %) auf das in einem Kessel rotierende Popcorn aufgetragen. Hierbei werden 10 - 15 g des Mediums / Auftrag aufgegeben. Dieses verteilt sich auf dem Popcorn während Trocknungsluft zugeleitet wird. Sobald ein Auftrag getrocknet ist, kann eine weitere Auftragsmenge auf das Popcorn aufgebracht werden, bis der gewünschte Farbeffekt erreicht ist. Im Anschluss wird das Popcorn, z.B. in einem Trockenschrank oder Ofen getrocknet, bis die Ausgangsfeuchtigkeit wieder erreicht wird.

### Beispiel 5.2: Popcorn gezuckert

| | | |
|---|---|---|
| Popcorn-Einwaage: | 300 g | |
| | | |
| Auftragsmedium: | 10 Gew.-% Candurin® Brown Amber | Merck KGaA |
| | 90 Gew.-% Auftragsmedium Basis (Zusammensetzung und Herstellung wie in Beispiel 4) | |
| | | |
| Auftragsmenge: | 60 g (= 20 %) | |

### Beispiel 6: Dragees (Schokolade, Zucker / zuckerfrei)

Das Perlglanzpigment-haltige Auftragsmedium wird analog zum herkömmlichen Dragieren per Hand auf die im Kessel rotierenden Schoko-Dragees aufgebracht. Auch hier werden Schichten nacheinander aufgetragen, bis der gewünschte Effekt erzielt ist. Sobald eine Schicht getrocknet ist, kann eine erneute Zugabe erfolgen. Ein geringer Auftrag ergibt einen Marmor-Effekt, während ein erhöhter Auftrag zu einer sehr gleichmäßigen Perlglanzfärbung führt.

Auftragsmedium (Basis):

| | | |
|---|---|---|
| 70 | Gew.-% Wasser | |
| 23 | Gew.-% Ethanol (96 vol-%) | Merck KGaA |
| 3,5 | Gew.-% 1,2-Propandiol E1520 | Merck KGaA |
| 2,0 | Gew.-% Natriumcarboxymethylcellulose | Roeper |
| 1,2 | Gew.-% Soja- bzw. Sonnenblumen-Lecithin | Sternchemie |
| 0,25 | Gew.-% Zitronensäure (kristallin) | Merck KGaA |
| 0,05 | Gew.-% Kaliumsorbat | Merck KGaA |

Optionale Zugabe von Süßstoffen und Aromen. Ethanol-Gehalt kann zur Rezepturanpassung variiert bzw. auch ganz durch Wasser und 1,2-Propandiol ersetzt werden.

### Beispiel 6.1: Haselnüsse, dragiert mit dunkler Schokolade

Dragees werden in einen Kessel gegeben. Dieser ist mit Mitnehmern ausgestattet. Trocknungsluft ist eingeschaltet (25 °C)

| | |
|---|---|
| Dragee-Einwaage: | 1.000 g |
| Auftragsmedium: | 10 Gew.-% Candurin® Silver Lustre Merck KGaA 90 Gew.-% Auftragsmedium Basis (Zusammensetzung wie in Beispiel 6, Herstellung wie in Beispiel 4) |
| | |
| Auftragsmenge: | 80 g (= 8%) |

### Beispiel 6.2: Schoko-Kaffeebohnen, dragiert mit Milchschokolade

### Herstellung wie in Beispiel 6.1

| | |
|---|---|
| Dragee-Einwaage: | 1.200 g |
| | |
| Auftragsmedium: | 10 Gew.-% Candurin® Brown Amber Merck KGaA 90 Gew.-% Auftragsmedium Basis (Zusammensetzung wie in Beispiel 6, Herstellung wie in Beispiel 4) |
| | |
| Auftragsmenge: | 95 g (= 7,9 %) |

### Beispiel 6.3: Rosinen, dragiert mit weißer Schokolade

### Herstellung wie in Beispiel 6.1

| | |
|---|---|
| Dragee-Einwaage: | 1.000 g |
| | |
| Auftragsmedium: | 8 Gew.-% Candurin® Gold Shimmer Merck KGaA 92 Gew.-% Auftragsmedium Basis (Zusammensetzung wie in Beispiel 6, Herstellung wie in Beispiel 4) |
| | |
| Auftragsmenge: | 60 g (= 6%) |

### Beispiel 6.4: Zuckerdragierte Schokolinsen, weiß

| | |
|---|---|
| Dragee-Einwaage: | 1.000 g |
| Auftragsmedium: | 8 Gew.-% Candurin® Brown Amber Merck KGaA 92 Gew.-% Auftragsmedium Basis (Zusammensetzung wie in Beispiel 6, Herstellung wie in Beispiel 4) |
| | |
| Auftragsmenge: | 100 g (= 10 %) |

### Beispiel 6.5: Isomalt-dragierte Kaugummikissen, weiß

| | | |
|---|---|---|
| Dragee-Einwaage: | 1.000 g | |
| | | |
| Auftragsmedium: | 8 Gew.-% Candurin® Brown Amber | Merck KGaA |
| | 92 Gew.-% Auftragsmedium Basis (Zusammensetzung wie in Beispiel 6, Herstellung wie in Beispiel 4) | |
| Auftragsmenge: | 80 g (= 8 %) | |

### Beispiel 6.6: Zuckerdragierte Mandeln, rot

| | | |
|---|---|---|
| Dragee-Einwaage: | 800 g | |
| Auftragsmedium: | 8 Gew.-% Candurin® Silver Lustre | Merck KGaA |
| | 92 Gew.-% Auftragmedium Basis (Zusammensetzung wie in Beispiel 6, Herstellung wie in Beispiel 4) | |
| | | |
| Auftragsmenge: | 72 g (= 9 %) | |

### Beispiel 6.7: Weitere Oberflächenoptimierung von Dragees

Sowohl bei den Schoko-dragierten Produkten (Beispiele 6.1 - 6.3), als auch bei den Zucker- bzw. zuckerfrei dragierten Produkten (Beispiele 6.4 - 6.6) kann der Perlglanz weiter verbessert werden, wenn nach dem Auftrag des Auftragsmediums und einer bestimmten Trockenzeit nochmals eine Mischung aus Schelllack, Ethanol und Perlglanzpigment aufgetragen wird. Idealerweise kann es sich hierbei um das bzw. die im Medium bereits enthaltenen Perlglanzpigmente handeln, doch sind auch andere Kombinationen möglich. Das Schelllack-Ethanol-Perlglanzpigment-Gemisch wird per Hand über die im Kessel rotierenden Dragees gegeben und mit Zuluft abgetrocknet. Alternativ kann die Mischung auch gesprüht werden.

Zusammensetzung (Beispiel):

| | |
|---|---|
| 90 Gew.-% | ethanolische Schellacklösung (2 - 50 %ig Schellackgehalt) (z.B. Capol® 425 oder Crystallac® (Fa. Mantrose-Haeuser Co., Inc.) |
| 10 Gew.-% | Perlglanzpigment (ausgewählt aus A1-A11 gemäß Beispiel 1) |

Vor der Zugabe des Perlglanzpigmentes kann die Schelllacklösung weiter mit Ethanol verdünnt werden (z.B. 1 Teil Schellacklösung verdünnt mit doppelter oder 5facher Menge Ethanol (Lebensmittelqualität) Auftragsmenge: 5-40 g/kg Produkt -je nach Verdünnung Perlglanz-Pigmentmenge im Schellacklösung (2-50 % Schellackgehalt) + 1 - 40% Pigment; vorzugsweise: 5 - 25% Pigment

### Beispiel 7: Färbung von Komprimaten

Eine direkte Färbung von Komprimaten mit Perlglanz-Pigmenten ist wenig sinnvoll, da zum einen der resultierende Perlglanz wenig sichtbar ist, und zum anderen hohe Pigment-Einsatzmengen erforderlich sind. Ein Aufsprühen erfordert meist lange Produktionszeiten und führt auch oft zu Beschädigungen der Komprimate während des Färbungsprozesses.

Mittels des erfindungsgemäßen Auftragsmediums kann eine gleichmäßige Färbung der Komprimate erreicht werden. Die Färbung erfolgt vorteilhaft in einem Dragierkessel und folgt dem in Beispiel 6 beschriebenen Prinzip. Ein weiterer Vorteil des erfindungsgemäßen Auftragsmediums besteht darin, dass es schnell eine Schutzschicht um die Komprimate bildet. Hierdurch wird das oft zu beobachtende "Rundlaufen" stark verringert. Mittels nur einigen wenigen Aufträgen kann man schnell einen interessanten "Marmor-Perlglanzeffekt" erzielen.

Falls gewünscht, können die Komprimate nach der Färbung in einem Ofen etc. auf die ursprüngliche Produktfeuchte getrocknet werden. Auftragsmedium (Basis):

| | | |
|---|---|---|
| 72 | Gew.-% Wasser | |
| 20 | Gew.-% Ethanol (96 Vol-%) | Merck KGaA |
| 4,5 | Gew.-% 1,2-Propandiol E1520 | Merck KGaA |
| 2,0 | Gew.-% Natriumcarboxymethylcellulose | Roeper |
| 1,2 | Gew.-% Soja- bzw. Sonnenblumen-Lecithin | Sternchemie |
| 0,25 | Gew.-% Zitronensäure (kristallin) | Merck KGaA |
| 0,05 | Gew.-% Kaliumsorbat | Merck KGaA |

Zur Rezepturanpassung können optional Süßstoffen und/oder Aromen zugegeben werden. Der Ethanol-Gehalt kann zur Rezepturanpassung variiert, bzw. Ethanol auch ganz durch Wasser und 1,2-Propandiol ersetzt werden.

### Beispiel 7.1: Pfefferminz-Komprimat, weiß

| | |
|---|---|
| Produkt-Einwaage: | 500 g |
| | |
| Perlglanzmedium: | 8 Gew.-% Candurin® Silver Sparkle, Merck KGaA |
| | 92 Gew.-% Auftragsmedium (Zusammensetzung wie in Beispiel 7, Herstellung wie in Beispiel 4) |
| | |
| Auftragsmenge: | 40 g (= 8 %) |

### Beispiel 7.2: Zitronen-Komprimat, hellgelb

| | |
|---|---|
| Produkt-Einwaage: | 500 g |
| | |
| Perlglanzmedium: | 6 Gew.-% Candurin® Gold Lustre Sparkle, Merck KGaA |
| | 94 Gew.-% Auftragsmedium Basis (Zusammensetzung wie in Beispiel 7, Herstellung wie in Beispiel 4) |
| | |
| Auftragsmenge: | 50 g (= 10 %) |

### Beispiel 8: Färbung, Dekoration von Marshmallows

Bei der Färbung von Marshmallows mit Perlglanzpigmenten werden bisher keine zufriedenstellende Ergebnisse erzielt. Durch die Aufbringung des erfindungsgemäßen Auftragsmediums lassen sich Perlglanzeffekte auf der Oberfläche von Marshmallows leicht erzielen.

Der Auftrag erfolgt in einem Dragierkessel. Der Prozess-Ablauf erfolgt wie in Beispiel 6 beschrieben.

Die Zusammensetzung des Mediums ist analog zu Komprimaten (Beispiel 7) aufgebaut. Gleiches gilt für mögliche Modifikationen desselben.

### Beispiel 8.1: Färbung von Marshmallows, weiß

| | | |
|---|---|---|
| Produkt-Einwaage: | 300 g | |
| | | |
| Perlglanzmedium: | 10 Gew.-% Candurin® Silver Sparkle | Merck KGaA |
| | 90 Gew.-% Auftragsmedium Basis (Zusammensetzung wie in Beispiel 7, Herstellung wie in Beispiel 4) | |
| | | |
| Auftragsmenge: | 51 g (= 17%) | |

### Beispiel 8.2: Färbung von Marshmallows, hellgrün

| | | |
|---|---|---|
| Produkt-Einwaage: | 300 g | |
| | | |
| Perlglanzmedium: | 10 Gew.-% Candurin® Gold Sparkle | Merck KGaA |
| | 90 Gew.-% Auftragmedium Basis (Zusammensetzung wie in Beispiel 7, Herstellung wie in Beispiel 4) | |
| | | |
| Auftragsmenge: | 51 g (= 17 %) | |

### Beispiel 8.3 Färbung von Marshmallows, rosa

| | | |
|---|---|---|
| Produkt-Einwaage: | 300 g | |
| Perlglanzmedium: | 8 Gew.-% | Candurin® Silver Sparkle Merck KGaA |
| | 92 Gew.-% | Basis (Zusammensetzung wie in Beispiel 7, Herstellung wie in Beispiel 4) |
| | | |
| Auftragsmenge: | 42 g (= 14 %) | |

### Beispiel 9: Herstellung von Dekor-Lace und Dekor-Blätter

Unter Dekor-Lace sind filigrane Formen und Dekore zu verstehen, welche durch das dünne Ausstreichen des erfindungsgemäßen Auftragsmediums in Silikon-Formen hergestellt werden. Diese werden nach einer bestimmten Trockenzeit aus den Formen entnommen und können zum Verzieren von z.B. Süßwaren, Eiskrem, Schokolade, Eiern, Pralinen, Backwaren, Kuchen und Torten, Desserts, Pates, Sülzen und anderer Lebensmittel verwendet werden. Die Trockenzeit kann hierbei so eingestellt werden, dass die Produkte noch eine gewisse Elastizität zeigen.

Auch ist eine Verwendung der hergestellten Dekore als essbarer Körperschmuck möglich. Hierbei werden die Dekore mittels geeigneter Haftmittel auf die entsprechenden Körperstellen aufgebracht. Weiterhin kann die Paste in deren Anwendung als essbare Kosmetik auch direkt auf zu färbende Körperstellen aufgetragen werden. Durch die Körperwärme erfolgt eine schnelle Abtrocknung auf der Haut.

Unter Dekor-Blättern versteht man sehr dünne, blätterförmige Strukturen, welche durch das sehr dünne Ausstreichen des Perlglanzmediums auf flachen Unterlagen hergestellt werden. Diese Unterlagen können z.B. aus Plastik oder Metall bestehen. Nach dem Ausstreichen werden die Produkte bis zur vollständigen Trocknung z.B. in einem Trockenschrank oder Ofen getrocknet. Nach dem Trocknen lassen sich die Dekor-Blätter von der Unterlage entfernen und können nun als Ganzes, teilweise oder entsprechend kleingeschnitten bzw. -gehackt zur Verzierung von Lebensmitteln, wie z.B. bei dem oben beschriebenen Dekor-Lace entsprechend aufgelistet, verwendet werden.

### Beispiel 9.1: Rezeptur Dekor-Lace:

| | | | |
|---|---|---|---|
| 76 | Gew.-% | Wasser | |
| 9,75 | Gew.-% | 1,2-Propandiol E1520 | Merck KGaA |
| 2,4 | Gew.-% | Natriumcarboxymethylcellulose | Roeper |
| 1,5 | Gew.-% | Soja- bzw. Sonnenblumen-Lecithin | Sternchemie |
| 0,3 | Gew.-% | Zitronensäure (kristallin) | Merck KGaA |
| 0,05 | Gew.-% | Kaliumsorbat | Merck KGaA |
| 10 | Gew.-% | Perlglanzpigment (ausgewählt aus A1) bis A11) gemäß Beispiel 1) | Merck KGaA |

Zur Rezepturanpassung können optional Süßstoffe und/oder Aromen zugegeben werden. Wasser- und 1,2-Propandiol-Anteil können variiert werden, um die Rezeptur kundenspezifisch anzupassen.

Ethanol kann ebenfalls hinzugegeben werden, um die Trocknung zu beschleunigen.

Nach der Herstellung wird das Auftragsmedium dünn in Silikon-Formen gestrichen und bei ca. 70 °C im Trockenschrank für 2 - 3 Stunden getrocknet. Danach können die Dekore vorsichtig aus der Form entnommen werden.

Färbungsmöglichkeiten des Auftragsmediums mit Perlglanzpigmenten, Kombinationen von Perlglanzpigmenten bzw. Kombinationen von Perlglanzpigmenten mit anderen natürlichen, mineralischen und künstlichen Lebensmittelfarbstoffen bzw. -pigmenten bestehen wie z.B. in Beispiel 1 angegeben.

### Beispiel 9.2: Rezeptur Dekorblätter

| | | | |
|---|---|---|---|
| 80 | Gew.-% | Wasser | |
| 5,75 | Gew.-% | 1,2 Propandiol E1520 | Merck KGaA |
| 2,4 | Gew.-% | Natriumcarboxymethylcellulose | Roeper |
| 1,5 | Gew.-% | Soja- bzw. Sonnenblumen-Lecithin | Sternchemie |
| 0,3 | Gew.-% | Zitronensäure (kristallin) | Merck KGaA |
| 0,05 | Gew.-% | Kaliumsorbat | Merck KGaA |
| 10 | Gew.-% | Perlglanzpigment (ausgewählt aus A1) bis A11) gemäß Beispiel 1) | Merck KGaA |

Zur Rezepturanpassung können optional Süßstoffe und/oder Aromen zugegeben werden. Wasser- und 1,2-Propandiol-Anteil können variiert werden, um die Rezeptur kundenspezifisch anzupassen.

Ethanol kann ebenfalls hinzugegeben werden, um die Trocknung zu beschleunigen.

Nach der Herstellung wird das Auftragsmedium sehr dünn auf geeignete Unterlagen (Plastik, Metall etc.) ausgestrichen, und im Anschluss im Trockenschrank oder Herd bei ca. 70 °C bis zur vollständigen Trocknung getrocknet. Danach können die Dekor-Blätter vollständig abgelöst werden und als Ganzes, teilweise bzw. kleingeschnitten als Dekorverzierungen bei Lebensmittelprodukten wie unter Dekor-Lace beschrieben verwendet werden.

Die in den Beispielen verwendeten Perlglanz- und Interferenzpigmente (Candurin®-Pigmente der Fa. Merck) basierend auf Glimmer- oder SiO₂-Plättchen besitzen folgende Zusammensetzung und Partikelgrößen:

| | |
|---|---|
| Candurin® Gold Lustre: | Glimmer (E555) beschichtet mit TiO₂ (E171) und Fe₂O₃ (E172ii); 10-60 µm |
| Candurin® Brown Amber: | Glimmer (E555) beschichtet mit Fe₂O₃ (E172ii); 10-60 µm |
| Candurin® Gold Shimmer: | Glimmer (E555) beschichtet mit TiO₂ (E171); 10-60 µm |
| Candurin® Silver Lustre: | Glimmer (E555) beschichtet mit TiO₂ (E171); 10-60 µm |
| Candurin® Silver Sparkle: | Glimmer (E555) beschichtet mit TiO₂ (E171); 10-150 µm |
| Candurin® Red Sparkle: | Glimmer (E555) beschichtet mit Fe₂O₃ (E172ii); 10-150 µm |
| | |
| Candurin® NXT Ruby Red: | SiO₂-Plättchen (E551) beschichtet mit Fe₂O₃ (E172ii); 5-50 µm |
| Candurin® Red Lustre: | Glimmer (E555) beschichtet mit Fe₂O₃ (E172ii); 10-60 µm |

## Patentansprüche

1. Verfahren zur Färbung von Oberflächen mit Effektpigmenten basierend auf plättchenförmigen Substraten, **dadurch gekennzeichnet, dass** (i) das Effektpigment in eine pastöse, transparente Masse auf Wasserbasis eingebracht und (ii) das in Schritt (i) hergestellte Auftragsmedium auf die Oberfläche aufgebracht wird.

2. Verfahren nach Anspruch 1, **dadurch gekennzeichnet, dass** die Oberfläche eine Lebensmittel-Oberfläche oder die Oberfläche des menschlichen Körpers ist.

3. Verfahren nach Anspruch 1 oder 2, **dadurch gekennzeichnet, dass** das Effektpigment ein Perlglanzpigment, ein Interferenzpigment oder ein Multilayerpigment ist.

4. Verfahren nach einem oder mehreren der Ansprüche 1 bis 3, **dadurch gekennzeichnet, dass** das plättchenförmige Substrat ein natürliches Glimmerplättchen, ein synthetisches Glimmerplättchen, Talkum, Kaolin, ein Glasplättchen, ein Siliziumdioxid-Plättchen, ein Titandioxid-Plättchen, ein Aluminiumoxid-Plättchen oder ein Eisenoxidplättchen ist.

5. Verfahren nach einem oder mehreren der Ansprüche 1 bis 4, **dadurch gekennzeichnet, dass** das plättchenförmige Substrat mit ein oder mehreren Schichten aus Metalloxiden und/oder Metalloxidgemischen vollständig beschichtet ist.

6. Verfahren nach einem oder mehreren der Ansprüche 1 bis 5, **dadurch gekennzeichnet, dass** das plättchenförmige Substrat mit Titandioxid und/oder Eisenoxid beschichtet ist.

7. Verfahren nach einem oder mehreren der Ansprüche 1 bis 6, **dadurch gekennzeichnet, dass** das Effektpigment ausgewählt ist der Gruppe der nachfolgend genannten Pigmente:
natürliche Glimmerplättchen + TiO₂
natürliche Glimmerplättchen + Fe₂O₃
natürliche Glimmerplättchen + Fe₃O₄
natürliche Glimmerplättchen + TiO₂ + Fe₂O₃
natürliche Glimmerplättchen + TiO₂ + Fe₃O₄
natürliche Glimmerplättchen + Fe₂O₃ + TiO₂
natürliche Glimmerplättchen + Fe₃O₄ + TiO₂
natürliche Glimmerplättchen + TiO₂/Fe₂O₃-Gemisch
natürliche Glimmerplättchen + TiO₂/Fe₃O₄-Gemisch
synthetische Glimmerplättchen + TiO₂
synthetische Glimmerplättchen + Fe₂O₃
synthetische Glimmerplättchen + Fe₃O₄
synthetische Glimmerplättchen + TiO₂ + Fe₂O₃
synthetische Glimmerplättchen + TiO₂ + Fe₃O₄
synthetische Glimmerplättchen + Fe₂O₃ + TiO₂
synthetische Glimmerplättchen + Fe₃O₄ + TiO₂
synthetische Glimmerplättchen + TiO₂/Fe₂O₃-Gemisch
synthetische Glimmerplättchen + TiO₂/Fe₃O₄-Gemisch
SiO₂-Plättchen + TiO₂
SiO₂-Plättchen + Fe₂O₃
SiO₂-Plättchen + Fe₃O₄
SiO₂-Plättchen + TiO₂ + Fe₂O₃
SiO₂-Plättchen + TiO₂ + Fe₃O₄
SiO₂-Plättchen + Fe₂O₃ + TiO₂
SiO₂-Plättchen + Fe₃O₄ + TiO₂
SiO₂-Plättchen + TiO₂/Fe₂O₃-Gemisch
SiO₂-Plättchen + TiO₂/Fe₃O₄-Gemisch
natürliche Glimmerplättchen + TiO₂ + SiO₂ + TiO₂
natürliche Glimmerplättchen + Fe₂O₃ + SiO₂ + TiO₂
natürliche Glimmerplättchen + TiO₂ + SiO₂ + Fe₂O₃
natürliche Glimmerplättchen + TiO₂ + SiO₂ + Fe₃O₄
natürliche Glimmerplättchen + TiO₂/Fe₂O₃-Gemisch + SiO₂ + Fe₂O₃
natürliche Glimmerplättchen + TiO₂/Fe₂O₃-Gemisch + SiO₂ + TiO₂/F e₂O₃-Gem isch
natürliche Glimmerplättchen + Fe₂O₃-Gemisch + SiO₂ + TiO₂/Fe₂O₃-Gemisch
synthetische Glimmerplättchen + TiO₂ + SiO₂ + TiO₂
synthetische Glimmerplättchen + Fe₂O₃ + SiO₂ + TiO₂
synthetische Glimmerplättchen + TiO₂ + SiO₂ + Fe₂O₃
synthetische Glimmerplättchen + TiO₂ + SiO₂ + Fe₃O₄
synthetische Glimmerplättchen + TiO₂/Fe₂O₃-Gemisch + SiO₂ + Fe₂O₃
synthetische Glimmerplättchen + TiO₂/Fe₂O₃-Gemisch + SiO₂ + TiO₂/F e₂O₃-Gem isch
synthetische Glimmerplättchen + Fe₂O₃-Gemisch + SiO₂ + TiO₂/F e₂O₃-Gem isch
SiO₂-Plättchen + TiO₂ + SiO₂ + TiO₂
SiO₂-Plättchen + Fe₂O₃ + SiO₂ + TiO₂
SiO₂-Plättchen + TiO₂ + SiO₂ + Fe₂O₃
SiO₂-Plättchen + TiO₂ + SiO₂ + Fe₃O₄
SiO₂-Plättchen + TiO₂/Fe₂O₃-Gemisch + SiO₂ + Fe₂O₃
SiO₂-Plättchen + TiO₂/Fe₂O₃-Gemisch + SiO₂ + TiO₂/Fe₂O₃-Gemisch
SiO₂-Plättchen + Fe₂O₃-Gemisch + SiO₂ + TiO₂/Fe₂O₃-Gemisch.

8. Verfahren nach einem oder mehreren der Ansprüche 1 bis 7, **dadurch gekennzeichnet, dass** 0,05 - 45 Gew.-% an Perlglanzpigment, bezogen auf die Gesamtmasse des Auftragsmediums, eingesetzt werden.

9. Verfahren nach einem oder mehreren der Ansprüche 1 bis 8, **dadurch gekennzeichnet, dass** (i) Effektpigmente einzeln, (ii) deren Mischungen oder (iii) Mischungen einzelner Effektpigmente mit weiteren Farbstoffen und/oder Pigmenten oder (iv) Mischungen von Mischungen von Perlglanzpigmenten mit weiteren Farbstoffen und/oder Pigmenten eingesetzt werden.

10. Verfahren nach einem oder mehreren der Ansprüche 1 bis 9, **dadurch gekennzeichnet, dass** zusätzlich Aromen und/oder Süßstoffe eingesetzt werden.

11. Verfahren nach einem oder mehreren der Ansprüche 1 bis 10, **dadurch gekennzeichnet, dass** die pastöse, transparente Masse zusätzlich mindestens eine Komponente ausgewählt aus der Gruppe Stabilisator, Feuchteregulator, Emulgator, Konservierungsstoff enthält.

12. Verfahren nach einem oder mehreren der Ansprüche 1 bis 11, **dadurch gekennzeichnet, dass** die pastöse, transparente Masse Wasser, mindestens einen Stabilisator, mindestens einen Feuchteregulator, mindestens einen Emulgator und mindestens einen Konservierungsstoff und optional weitere Komponenten enthält.

13. Verfahren nach einem oder mehreren der Ansprüche 1 bis 12, **dadurch gekennzeichnet, dass** der Stabilisator in einer Menge von 0,25 - 8 Gew.-%, bezogen auf die Gesamtmasse des Auftragsmediums, eingesetzt wird.

14. Verfahren nach einem oder mehreren der Ansprüche 1 bis 13, **dadurch gekennzeichnet, dass** der Feuchteregulator in einer Menge von 0,5 - 25 Gew.-%, bezogen auf die Gesamtmasse des Auftragsmediums, eingesetzt wird.

15. Verfahren nach einem oder mehreren der Ansprüche 1 bis 14, **dadurch gekennzeichnet, dass** der Emulgator in einer Menge von 0,25 - 5 Gew.-%, bezogen auf die Gesamtmasse des Auftragsmediums, eingesetzt wird.

16. Verfahren nach einem oder mehreren der Ansprüche 1 bis 15, **dadurch gekennzeichnet, dass** der Konservierungsstoff in einer Menge von 0,1 - 1,5 Gew.-%, bezogen auf die Gesamtmasse des Auftragsmediums, eingesetzt wird.

17. Verfahren nach einem oder mehreren der Ansprüche 1 bis 16, **dadurch gekennzeichnet, dass** das Auftragsmedium zusätzlich Ethanol enthält.

18. Verfahren nach einem der Ansprüche 1 bis 17, **dadurch gekennzeichnet, dass** das Auftragsmedium zunächst mittels entsprechender Formen oder Unterlagen in eine gewünschte Form gebracht und danach auf die Oberfläche aufgebracht wird.

19. Masse enthaltend Wasser, mindestens einen Stabilisator, mindestens einen Feuchteregulator, mindestens einen Emulgator, mindestens einen Konservierungsstoff und gegebenenfalls weitere Komponenten.

20. Medium zum Auftrag von Effektpigmenten basierend auf plättchenförmigen Substraten auf Oberflächen, **dadurch gekennzeichnet, dass** das Medium ein oder mehrere Effektpigmente sowie die Masse gemäß Anspruch 19 enthält.

21. Mit Medium gemäß Anspruch 20 versehene Oberfläche.

22. Mit Medium gemäß Anspruch 21 versehene Oberfläche, wobei die Oberfläche die Oberfläche von Lebensmitteln oder die der Haut des menschlichen Körpers ist.
